# EUROPÄISCHE PATENTSCHRIFT

(11) **EP 3 305 908 B1**
(45) Veröffentlichungstag und Bekanntmachung des Hinweises auf die Patenterteilung: **14.08.2019**
(21) Anmeldenummer: 16192488.1
(22) Anmeldetag: 05.10.2016
(51) Int. Cl.: C12Q 1/04, C12N 1/20

(54) **FLÜSSIGES NÄHRMEDIUM FÜR DIE KULTIVIERUNG KÄSEREISCHÄDLICHER CLOSTRIDIEN UND VERFAHREN ZU DEREN NACHWEIS**
LIQUID NUTRIENT MEDIUM FOR THE CULTIVATION OF CLOSTRIDIA WHICH IS HAZARDOUS FOR THE CHEESE-MAKING INDUSTRY AND DETECTION OF SAME
MILIEU DE CULTURE LIQUIDE POUR LA CULTURE DE CLOSTRIDIUM PATHOGÈNE ET PROCÉDÉ POUR LE DÉTECTEUR

(43) Veröffentlichungstag der Anmeldung: 11.04.2018
(73) Patentinhaber: SY-LAB Vertriebsgesellschaft m.b.H., 3011 Neupurkersdorf (AT)
(72) Erfinder: Brändle, Johanna, 1040 Wien (AT); Domig, Konrad, 1200 Wien (AT); Schinkinger, Manfred, 3550 Langenlois (AT); Stocker, Wolfgang, 2721 Bad Fischau (AT)
(74) Vertreter: Schwarz & Partner Patentanwälte OG

(56) Entgegenhaltungen:
- EP-A2- 3 190 173
- WO-A1-2015/184454
- JP-A- 2006 304 698
- ANDERS JONSSON: "Enumeration and Confirmation of Clostridium tyrobutyricum in Silages Using Neutral Red, D-Cycloserine, and Lactate Dehydrogenase Activity", JOURNAL OF DAIRY SCIENCE., Bd. 73, Nr. 3, 1. März 1990 (1990-03-01), Seiten 719-725, XP055349159, US ISSN: 0022-0302, DOI: 10.3168/jds.S0022-0302(90)78725-5
- LAURENCE GIRBAL ET AL: "How neutral red modified carbon and electron flow in Clostridium acetobutylicum grown in chemostat culture at neutral pH", FEMS MICROBIOLOGY REVIEWS, Bd. 16, 1. Januar 1995 (1995-01-01), Seiten 151-162, XP055351956,
- M R POPOFF: "Selective medium for isolation of Clostridium butyricum from human feces", JOURNAL OF CLINICAL MICROBIOLOGY, Bd. 20, Nr. 3, 1. September 1984 (1984-09-01), Seiten 417-420, XP055352382, US ISSN: 0095-1137
- LEO KAUFMAN ET AL: "USE OF NEUTRAL RED FLUORESCENCE FOR THE IDENTIFICATION OF COLONIES OF CLOSTRIDIA", J BACTERIOL., Bd. 79, Nr. 2, 1. Februar 1960 (1960-02-01), Seite 292, XP055349168,
- BRÄNDLE J ET AL: "Relevance and analysis of butyric acid producing clostridia in milk and cheese", FOOD CONTROL, BUTTERWORTH, LONDON, GB, Bd. 67, 27. Februar 2016 (2016-02-27), Seiten 96-113, XP029492467, ISSN: 0956-7135, DOI: 10.1016/J.FOODCONT.2016.02.038

## Beschreibung

Die Erfindung betrifft ein flüssiges Nährmedium für die Kultivierung käsereischädlicher Clostridien sowie ein Verfahren für den selektiven Nachweis von buttersäureproduzierenden anaeroben Sporenbildnern der Gattung Clostridium in Milch und Käse.

### HINTERGRUND

Wenn Käsereimilch mit Clostridiensporen belastet ist, so entsteht während der Reifung von Hartkäse durch das Auskeimen dieser Sporen ein charakteristischer Käsefehler, die sogenannte Spätblähung. Die Gruppe der käsereischädlichen Clostridien umfasst insbesondere die buttersäurebildenden Clostridien. Die Anwesenheit der ranzig riechenden Buttersäure und die starke Gasentwicklung resultieren in einer unerwünschten Sensorik, in Rissbildungen und unregelmäßigen Lochungen und führen zu hohen wirtschaftlichen Verlusten. Da bereits einige wenige Sporen pro Liter Rohmilch diese Käsefehler verursachen können, ist ein verlässlicher quantitativer Nachweis essentiell für die Käseerzeugung. Die genaue Erfassung der Sporenzahlen ist darüber hinaus auch in jenen Gegenden von Bedeutung, in denen die Sporenzahl in der Rohmilch als Qualitätsparameter gilt und somit Einfluss auf den Milchpreis hat. Weiters kann über die Anzahl der anaeroben Sporenbildner in der Rohmilch eine Aussage über die Art des verwendeten Rauhfutters (Heu, Gras bzw. Silage) getroffen werden.

Buttersäure produzierende Clostridien gehören zu der großen und sehr heterogenen Gattung *Clostridium* (Blaschek, 1999). Die buttersäureproduzierenden Clostridien werden dabei einem eigenen Cluster, Cluster 1, *Clostridium senso stricto* zugeordnet (Rainey, 2009). Es handelt sich dabei um gram-positive Stäbchenbakterien, die nur unter strikt anaeroben Bedingungen wachsen können. Der G+C-Gehalt ihrer Nukleinsäuren variiert zwischen 22 und 35%, und die Ähnlichkeit in ihren 16s rRNA-Sequenzen beträgt zwischen 92 und 99% (Rainey 2009). Sie sind in der Lage, resistente Endosporen zu produzieren, welche weder durch extreme Temperaturen (Hitze und Kälte) noch Strahlung oder extreme pH-Werte inaktiviert werden können. Diese Mikroorganismen können außerdem viele verschiedene Habitate mit starken jahreszeitlichen Schwankungen besiedeln und sie finden darüber hinaus ideale Vermehrungsbedingungen in siliertem Gras. Clostridien passieren den Verdauungstrakt von Wiederkäuern unbeschadet, weshalb die Kontamination der Rohmilch in aller Regel via fäkaler Verunreinigung erfolgt.

Bei jenen 4 Species, die am häufigsten aus Milch und Käse isoliert worden sind, handelt es sich um *Clostridium butyricum, Clostridum sporogenes, Clostridum beijerinckii* und *Clostridium tyrobutyricum,* wobei *C. tyrobutyricum* als Hauptverursacher der Käsefehler beschrieben worden ist (Zangerl 1993, Julien et.al. 2008).

Damit auch geringe Mengen an Käsereischädlingen überhaupt nachgewiesen werden können, erfolgt derzeit der Nachweis über das "Most Probable Number" (MPN)-Verfahren. Das MPN-Verfahren versucht, auf statistischem Weg durch mehrfachen Ansatz der Einwaagen die "wahrscheinlichste Keimzahl" zutreffend zu bestimmen. Dabei werden von jeder Einwaage mehrere Replikate parallel untersucht. Mit steigender Zahl der Parallelen nimmt dabei die Genauigkeit zu. Die Probe wird dazu meist in einer Reihe von Dezimalschritten verdünnt und von jeder dieser seriellen Verdünnungen werden 3 bis 5 Replikate in einer entsprechenden Nährlösung überimpft und bebrütet. Die Nährlösungen werden mit einem Paraffinpfropfen überschichtet und anaerob mindestens 3 bis 5 Tage bebrütet. Wachstum wird danach über die Gasbildung und das Aufsteigen des Paraffinpfropfens festgestellt. Aus der Anzahl der positiven Röhrchen in jeder der drei Verdünnungsstufen ergibt sich eine Zahlenkombination (=Stichzahl), der man mit statistischen Methoden eine "wahrscheinlichste Keimzahl" (MPN) pro Liter Probe zuordnen kann. Die MPN - Stichzahl kann dabei einfach über entsprechende statistische Tabellen (MPN-Tabellen) in die wahrscheinlichste Keimzahl (Sporenzahl) überführt werden. (Bergere J. and Sivelä S. ,1990).

Von Brändle et.al. (2016) werden die aktuell verfügbaren und für den Nachweis von käsereischädlichen Clostridien verwendeten Nährmedien im Detail beschrieben. Am häufigsten kommen dabei Rezepturen zum Einsatz, die auf der Verwendung des von Hirsch und Grinstead (1954) entwickelten "Reinforced Clostridial Medium" (RCM) basieren. RCM enthält im Wesentlichen die folgenden Komponenten:
Trypton bzw. Peptron
Fleischextrakt
Hefeextrakt
Stärke
Glucose
Natriumacetat
Cystein (gewöhnlich in Form von Cystein-HCl)
Natriumchlorid

All diese für den Nachweis von käsereischädlichen Clostridien verwendeten Nährmedien nutzen meist einen Laborpasteurisationsschritt, um die nicht sporenbildende Begleitflora zu eliminieren. Weiters wird häufig Laktat zugesetzt, um das Wachstum der käsereischädlichen Clostridien zu fördern. Trotz dieser Vorkehrungen und einer strikt anaeroben Bebrütung können aber auch andere in der Probe vorhandene, thermostabile und unter anaeroben Bedingungen vermehrungsfähige Mikroorganismen anwachsen und das Vorhandensein von Käsereischädlingen vortäuschen und hierdurch das Ergebnis verfälschen. Weil keines der bekannten Nährmedien eine ausreichende Sicherheit für den verlässlichen und vergleichbaren Nachweis gewährleistet, war es bisher jedoch nicht möglich, eine entsprechende Standardmethode für den Nachweis zu entwickeln.

Die derzeit verfügbaren Methoden für den Nachweis von käsereischädlichen Clostridien sind darüber hinaus auch extrem arbeits- und zeitintensiv. Nach der MPN-Methode müssen beispielsweise mindestens 3 sequentielle Probenverdünnungen in jeweils 5 Replikaten untersucht werden, wobei die Ergebnisse in der Regel erst nach 3 bis 10 Tagen Bebrütung vorliegen.

Die Erfindung versucht, die oben genannten Probleme zu beseitigen sowie die Nachteile des Standes der Technik zu überwinden, und stellt sich die Aufgabe, ein Nährmedium der eingangs genannten Art bereitzustellen, mit dem eine einfachere Auswertung der Ergebnisse ermöglicht wird und die Nachweiszeit verkürzt werden kann. Außerdem soll das Nährmedium in der Lage sein, eine Minimierung von falschen Positivergebnissen zu erreichen.

Eine weitere Aufgabe der Erfindung besteht darin, ein Verfahren zum Nachweis von käsereischädlichen Clostridiensporen in Milchproben nach der "Most Probable Number" (MPN) - Methode bereitzustellen, welches mithilfe des neuen Nährmediums für eine schnellere und zuverlässigere Quantifizierung der Clostridiensporen sorgt.

### KURZFASSUNG

Die oben genannte erste Aufgabe wird durch ein flüssiges Nährmedium für die Kultivierung käsereischädlicher Clostridien, enthaltend ein Reinforced Clostridial Medium (RCM) zur Inkubation mit Milch- oder Käseproben gelöst, welches Neutralrot als Indikator sowie Cycloserin und Trimethoprim umfasst.

Ein Farbindikatorsystem auf Basis des Redoxindikators Neutralrot, den die buttersäurebildenden Clostridien reduzieren können und der dabei die Farbe von Rot nach Gelb ändert, vereinfacht die Feststellung des Wachstums der Zielorganismen in flüssigen Nährmedien und erleichtert wesentlich die Auswertung, die derzeit nur über die Gasbildung bzw. das Aufsteigen des Paraffinpropfens, mit dem die Probe überschichtet wird, erfolgt.

Vorzugsweise beträgt die Konzentration von Neutralrot bei der Inkubation 0,010 bis 0,025 g/l, vorzugsweise 0,015 g/l.

Das Nährmedium enthält eine Kombination der Antibiotika Cycloserin und Trimethoprim, die sich, wie nachstehend in den Beispielen gezeigt wird, dazu eignen, das Wachstum der unerwünschten Begleitflora wie Bacillus spp. und Paenibacillus spp. zu unterdrücken und gleichzeitig die Zielorganismen, wie Cl. tyrobutyricum, Cl. butyricum, Cl. Sporogenes, zu besserem Wachstum zu stimulieren.

Gemäß einer bevorzugten Ausführungsform beträgt die Konzentration von Trimethoprim bei der Inkubation 17 mg/l bis 35 mg/l, vorzugsweise 28 mg/l. Eine andere bevorzugte Ausführungsform des erfindungsgemäßen Nährmediums ist dadurch gekennzeichnet, dass die Konzentration von Cycloserin bei der Inkubation 200 mg/l beträgt.

Primäres Ziel der vorliegenden Erfindung war es zudem, für den spezifischen Nachweis von anaeroben Sporenbildnern in Milch und Milchprodukten ein Verfahren und insbesondere auch für dieses Verfahren ein optimiertes Kulturmedium zu entwickeln, das den sensitiven Nachweis auch geringer Sporenmengen unter gleichzeitiger optimaler Unterdrückung der Begleitflora ermöglicht.

Eine weitere Aufgabe der Erfindung wird bei einem Verfahren zum Nachweis von käsereischädlichen Clostridien in Milch nach der "Most Probable Number" (MPN) - Methode unter Verwendung des erfinderischen flüssigen Nährmediums, wobei die Milch mit dem Nährmedium inkubiert wird, dadurch gelöst, dass die Inkubation der Milch bei einem pH-Wert von 6,0-7,0, vorzugsweise 7,0, unter anaeroben Bedingungen durchgeführt und die Probe nach der Inkubation hinsichtlich einer Farbänderung ausgewertet wird.

In einer bevorzugten Ausführungsform wird das Nährmedium dabei mit der Milch gemischt, wobei der Anteil der Milch >50 Vol.%, zum Beispiel 55%, 60%, 65%, 70%, 75%, 80%, 85%, besonders bevorzugt 75%, beträgt.

Das modifizierte Nährmedium enthält dabei die zu untersuchende Milch sozusagen als flüssigen Hauptbestandteil und die aus dem RCM-Medium bekannten Nährstoffe sowie den Indikator (und gegebenenfalls die Antibiotikakombination Cycloserin + Trimethoprim) zur maximalen Stimulation des Wachstums der buttersäureproduzierenden Clostridien bei Inkubation unter Sauerstoffausschluss.

Nach einer anderen bevorzugten Ausführungsform wird die Inkubation mittels mehrfacher Replikate von seriellen Probenverdünnungen in Mikrotiterplatten durchgeführt und die Auswertung mittels Fluoreszenzmessung vorgenommen, wobei das Nährmedium mit der Milch in einem Volumenverhältnis Nährmedium:Milch = 9:1 oder größer gemischt wird. In dieser Ausführungsform wird der "Milchbestandteil" des Nährmediums vergleichsweise gering gehalten, um die Messung des Indikatorfluoreszenzsignals zu gewährleisten.

### DETAILLIERTE BESCHREIBUNG

Das Nährmedium für den Nachweis von buttersäureproduzierenden Clostridien besteht erfindungsgemäß aus den RCM-Nährstoffen Trypton, Fleischextrakt, Hefeextrakt, Stärke, Glucose, Natriumacetat, Cystein und Natriumchlorid (in den an sich literaturbekannten und handelsüblichen Konzentrationen bzw. Konzentrationsbereichen) sowie Neutralrot, zumeist in Form eines flüssigen Konzentrats. Die Nährstoffkomponenten können auch durch vergleichbare Nährstoffe (in vergleichbaren Konzentrationen) ersetzt werden.

Werden beim Nachweisverfahren größere Mengen der Untersuchungsprobe, d.h. Milch oder mit UHT-Milch versetzter Probe (Käseisolat), zur Inkubation mit dem Nährmedium verwendet, hat der Probenansatz eine starke Eigentrübung und die Feststellung einer Trübung kann nicht als Indikator für das Anwachsen der Mikroorganismen herangezogen werden. Wird auch auf eine Überschichtung der Probensuspension mit Paraffin verzichtet, kann die Gasbildung, die mit der Verschiebung des Paraffinpfropfens einhergeht, ebenfalls nicht als Wachstumsindikator verwendet werden.

Der erfindungsgemäß eingesetzte Redoxindikator Neutralrot wird hingegen von Clostridien reduziert (Jonsson (1990) und kann daher sowohl über eine einfache Farbänderung von Rot nach Gelb als auch über ein Fluoreszenzsignal (Anregung bei 450-470 nm und Emission bei 550-590 nm) als Nachweis für das Anwachsen der Zielkeime verwendet werden.

Bei der Verwendung von >50 Vol.%, also z.B. 3 Teilen, der Untersuchungsprobe zur Inkubation mit dem Nährmedium ist beim erfindungsgemäßen Indikatorsystem der Nachweis nur über die Farbänderung möglich. Die Option der Fluoreszenzdetektion ist aufgrund der Eigenfluoreszenz der Probe (Milch) ausschließlich bei Verwendung kleinerer Probenmengen, also wenn das Nährmedium mit der Probe in einem größeren Volumenverhältnis, d.h. 9:1 oder größer, gemischt wird, möglich.

Die für den Nachweis von Clostridien im MPN-Verfahren derzeit verwendeten flüssigen Nährmedien basieren auf dem oben beschriebenen RCM-Medium, welches die grundlegenden, für die Anzucht benötigten Wachstumskomponenten enthält. Unerwünschte Begleitflora wird bei den gängigen, auf RCM basierenden Verfahren durch eine Laborpasteurisation bei 80 - 85°C und die aerobe Begleitflora zusätzlich durch eine Bebrütung unter Ausschluss von Sauerstoff durch Überschichten der Nährmedien mit Paraffin unterdrückt. Beide Maßnahmen reichen aber in der Regel nicht aus, um die unerwünschte Begleitflora wirksam zu unterdrücken, und die ermittelten Sporenzahlen werden, bedingt durch das Durchwachsen der Begleitflora, häufig verfälscht.

In zahlreichen Arbeiten wurden bereits Versuche beschrieben, bei denen durch Zusatz verschiedener Antibiotika das Wachstum der unerwünschten Begleitflora unterdrückt wurde. Ohne Anspruch auf Vollständigkeit in der Aufzählung kamen dabei die Antibiotika Polymyxin B, Sulfadizin, Cycloserin und Trimethoprim, zumeist in Kombination von 3 bis 4 der genannten Komponenten, zum Einsatz (Abgrall. et al (1985), Bourgeois et al. (1984), Batina et al. (1997), Jonsson (1990), Roux and Raoult (2004)). Dabei hat sich aber auch gezeigt, dass es meist zu einer unerwünschten Hemmung der nachzuweisenden Clostridienkultur selbst kam.

Die Kombination von Cycloserin und Trimethoprim kommt als bevorzugte Kombination der Selektivkomponenten des Nährmediums zur Unterdrückung der Begleitflora, im Wesentlichen Bacillus spp. und Paenibacillus spp., zum Einsatz. Die Kombination dieser Substanzen hat dabei keinen nachteiligen Einfluss auf das Wachstum der Zielorganismen. Es wurde sogar festgestellt, dass durch den Zusatz von Trimethoprim im bevorzugten Konzentrationsbereich das Wachstum der Zielorganismen überraschenderweise noch beschleunigt wird. Die diesbezüglichen Ergebnisse sind in Tabelle 1 zusammengefasst. Wie aus dieser Tabelle ersichtlich, konnten die Nachweiszeiten für die Zielorganismen (Cl. tyrobutyricum, Cl. butyricum, Cl. sporogenes und Cl. beijerinkii) deutlich und teilweise bis um die Hälfte reduziert werden.

**Tabelle 1: Vergleich der Detektionszeit (Zeit bis zum Auftreten eines sichtbaren Farbumschlags) während der Bebrütung mit bzw. ohne Zusatz von Trimethoprim.**

| **Code** | **Spezies** | **Stamm** | **ohne Trimethoprim** | **mit Trimethoprim** |
|---|---|---|---|---|
| | | | **Arithm. Mittelwert [h]** | |
| **Cl 2** | *Cl. sporogenes* | Uni Graz 8260 | 30.8 | 15.0 |
| **Cl 3** | *Cl. tyrobutyricum* | NCDO 1759 | 43.0 | 33.0 |
| **Cl 9** | *Cl. sporogenes* | LMG 8421; ATCC 3584; DSM 795 | 37.0 | 19.0 |
| **Cl 12** | *Cl. sporogenes* | ATCC 19404 | 15.9 | 12.5 |
| **Cl 13** | *Cl. beijerinckii* | IMB 4132 202 | 28.5 | 21.5 |
| **Cl 14** | *Cl. tyrobutyricum* | DSM 663 | 31.6 | 21.5 |
| **Cl 15** | *Cl. tyrobutyricum* | DSM 664 | 24.5 | 20.0 |
| **Cl 16** | *Cl. tyrobutyricum* | IMB 4132 2020 | 35.0 | 23.0 |
| **Cl 17** | *Cl. butyricum* | DSM 2477 4P1 | 15.3 | 7.5 |
| **Cl 18** | *Cl. butyricum* | DSM 2478 MMP3 | 24.8 | 19.0 |
| **Cl 19** | *Cl. butyricum* | DSM 10702; ATCC 19398 | 11.0 | 8.0 |
| **Cl 20** | *Cl. tyrobutyricum* | DSM 2637 | 45.7 | 41.0 |
| **Cl 22** | *Cl. beijerinckii* | IMB 4132 2008 | 28.5 | 12.5 |
| **Cl 24** | *Cl. beijerinckii* | IMB 4132 2007 | 17.8 | 12.0 |
| **Cl 25** | *Cl. tyrobutyricum* | IMB 4132 2022 | 46.8 | 28.0 |
| **Cl 26.2** | *Cl. beijerinckii* | IMB 4132 2023 | - | 31.5 |
| **Cl 29.2** | *Cl. tyrobutyricum* | IMB 4132 2010 | 30.0 | 28.0 |
| **Cl 31** | *Cl. beijernickii* | IMB 4132 2014 | 35.5 | 29.0 |
| **Cl 33** | *Cl. tyrobutyricum* | IMB 4132 2018 | 40.5 | 26.0 |
| **Cl 34** | *Cl. beijerinckii* | DSM-1820 | 31.5 | 24.0 |
| **Cl 36** | *Cl. beijerinckii* | DSM-791 | 35.3 | 25.0 |
| **Cl 37** | *Cl. acetobutylicum* | LMG 5710; DSM 792 | - | 37.5 |
| **Cl 40** | *Cl. beijerinckii* | LMG 1219; ATCC 6015 | 28.3 | 22.5 |
| **Cl 201** | *Cl. beijerinckii* | IMB 4132 201 | 28.5 | 14.5 |
| **Cl 1904** | *Cl. sporogenes* | IMB 4132 1904 | 19.3 | 10.0 |
| **Cl 2002** | *Cl. beijerinckii* | IMB 4132 2002 | 26.8 | 18.0 |
| **Cl 2013** | *Cl. tyrobutyricum* | IMB 4132 2009 | 29.0 | 24.0 |
| **Cl 2016** | *Cl. beijerinckii* | IMB 4132 2016 | 18.0 | 13.0 |
| **Cl 2021** | *Cl. tyrobutyricum* | IMB 4132 2021 | 18.0 | 14.5 |
| **I 18** | *Cl. tyrobutyricum* | Käseisolat | 27.8 | 19.0 |
| **I 19** | *Cl. tyrobutyricum* | Käseisolat | 25.0 | 19.0 |
| **I 20** | *Cl. tyrobutyricum* | Käseisolat | 36.0 | 19.0 |
| **I 22** | *Cl. tyrobutyricum* | Käseisolat | 26.5 | 18.0 |
| **I 23** | *Cl. tyrobutyricum* | Käseisolat | 31.5 | 19.0 |
| **I 52** | *Cl. tyrobutyricum* | Käseisolat | 31.5 | 19.0 |
| **I 53** | *Cl. tyrobutyricum* | Käseisolat | 31.5 | 22.0 |
| **I 54** | *Cl. tyrobutyricum* | Käseisolat | 31.5 | 19.0 |
| **I 56** | *Cl. tyrobutyricum* | Käseisolat | 30.0 | 19.0 |
| **I 57** | *Cl. tyrobutyricum* | Käseisolat | 40.5 | 37.0 |
| **I 62** | *Cl. tyrobutyricum* | Käseisolat | 42.0 | 28.0 |
| **I 63** | *Cl. tyrobutyricum* | Käseisolat | 42.0 | 35.5 |
| **K 6.5** | *Cl. tyrobutyricum* | Käseisolat | 48.0 | 27.0 |
| **K 8.1** | *Cl. tyrobutyricum* | Käseisolat | 30.8 | 19.0 |
| **K 11.2** | *Cl. tyrobutyricum* | Käseisolat | 27.0 | 27.0 |
| **K 13.2** | *Cl. tyrobutyricum* | Käseisolat | 31.5 | 24.5 |
| **K 13.5** | *Cl. tyrobutyricum* | Käseisolat | 31.5 | 19.0 |
| **K 16.3** | *Cl. tyrobutyricum* | Käseisolat | 27.0 | 19.0 |
| **K 18.1** | *Cl. tyrobutyricum* | Käseisolat | 22.5 | 18.0 |
| **K 18.2** | *Cl. tyrobutyricum* | Käseisolat | 29.3 | 24.5 |
| **K 25.1** | *Cl. tyrobutyricum* | Käseisolat | 46.0 | 39.0 |
| **K 26.1** | *Cl. tyrobutyricum* | Käseisolat | 37.0 | 22.0 |
| **K 27.1** | *Cl. tyrobutyricum* | Käseisolat | 37.0 | 27.5 |
| **K 27.9** | *Cl. tyrobutyricum* | Käseisolat | 39.0 | 24.0 |
| **K 28.5** | *Cl. tyrobutyricum* | Käseisolat | 33.5 | 24.0 |

Es wurde darüber hinaus festgestellt, dass Trimethoprim zur Unterdrückung von Paenibacillus thermophilus geeignet ist, welcher in einer Vielzahl von untersuchten Proben aus Rohmilchkäsen vorkommt und zu falsch positiven Ergebnissen in der Methode nach Bryant und Burkey führen kann. Wie nachfolgend gezeigt wird, konnte Trimethoprim in den Konzentrationen 17,5µg/ml, 28 µg/ml sowie 35µg/ml das Wachstum von Paenibacillus thermophilus sowie Paenibacillus barengoltzii unterdrücken und hatte keinen nachteiligen Einfluß auf die Zielorganismen. (Tabelle 2)

**Tabelle 2: Ergebnisse der erfindungsgemäßen Nährmedienzusammensetzung bei verschiedenen Trimethoprimkonzentrationen**

| **Isolat** | **Wachstum mit Farbumschlag Trimethoprimkonzentration** | | | |
|---|---|---|---|---|
| | 0 | 17,5µg/ml | 28 µg/ml | 35µg/ml |
| Clostridium tyrobutyricum | + | + | + | + |
| Clostridium butyricum | + | + | + | + |
| Paenibacillus thermophilus | + | - | - | - |
| Paenibacillus barengoltzii | + | - | - | - |
| Paenibacillus peoriae | - | - | - | - |

Das Nachweisverfahren beruht auf einer Inkubation einer Serie von Replikaten von seriellen Probenverdünnungen in der bereitgestellten Nährmedienzusammensetzung über einen bestimmten Zeitraum, um das Anwachsen der Zielkeime zu ermöglichen. Wie von den Erfindern festgestellt wurde, reicht beim erfindungsgemäßen Verfahren eine Inkubationszeit von 48 Stunden unter strikt anaeroben Bedingungen in einem Gemisch von 80% CO₂, 10% N₂ und 10% H₂ aus, um im Falle des Anwachsens der Zielkeime den im Medium enthaltenen Redoxinkubator von Rot nach Gelb umschlagen zu lassen und somit das Wachstum der Zielkeime nachzuweisen. Zur Erzielung von anaeroben Bedingungen können auch kommerziell erhältliche Gas-Generation-Kits in entsprechenden Bebrütungssystemen eingesetzt werden.

Die Farbänderung kann ohne entsprechende Apparate mit freiem Auge oder aber durch entsprechende fotooptische Auswertesysteme festgestellt werden. Für die Messung der Fluoreszenzsignale ist ein Fluoreszenzauswertesystem erforderlich.

Erfindungsgemäß wird das Nachweisverfahren bei einem End-pH-Wert des Nährmediums (inklusive der Untersuchungsprobe) von 6,0 bis 7,2, vorzugsweise 6,5 bis 7,2, am meisten bevorzugt bei pH 7,0, durchgeführt.

Durch das erfindungsgemäße Nachweisverfahren ist es möglich, Sporenzahlen von 75 bis 59.000 Sporen pro Liter Milch verlässlich nachzuweisen. Durch Einsatz entsprechender Mikrotitersysteme kann die Nachweisgrenze des Verfahrens sogar auf <30 Sporen pro Liter reduziert werden.

Die Erfindung wird nachfolgend anhand von Beispielen bevorzugter Ausführungsformen näher erläutert.

### BEISPIELE

Ein bevorzugtes Beispiel für eine Zusammensetzung des erfindungsgemäßen Nährmediums ist in Tabelle 3 angeführt:

**Tabelle 3: Zusammensetzung Nährmedium (als 4-fach Konzentrat)**

| **Basismedium (RCM)** | **Konzentration** |
|---|---|
| Trypton | 20g/l |
| Fleischextrakt | 40g/l |
| Hefeextrakt | 12 g/l |
| Stärke | 4 g/l |
| D(+) Glucose | 20 g/l |
| Na-Acetat | 12 g/l |
| Cystein-HCl | 2 g/l |
| NaCl | 20 g/l |
| | |

| **Zusätze** | |
|---|---|
| Neutralrot | 0,060 g/l |
| D-Cycloserin | 800 mg/l |
| Trimethoprim | 112 mg/l |

Für die Herstellung des erfindungsgemäßen Nährmediums bzw. für das erfindungsgemäße Verfahren kann auch ein handelsübliches RCM-Medium verwendet werden.

### Herstellung des Nährmediums (Untersuchungsmedium):

Die Komponenten werden mit Ausnahme von Cycloserin und Trimethoprim in beliebiger Reihenfolge eingewogen und in destilliertem Wasser gelöst. Der pH-Wert wird mit 5 M Natronlauge auf pH=8,0 eingestellt und das Basismedium (RCM) + Indikator (Neutralrot) 15 min bei 121°C autoklaviert. Nach dem Autoklavieren beträgt der pH-Wert 7,3 und erreicht nach Zugabe der Probe (3 Teile Milch) den für die Untersuchung vorgesehenen pH-Wert von 7,0.

Trimethoprim und D-Cycloserin werden als Stocklösung hergestellt und dem Basismedium + Indikator erst vor der Verwendung zugegeben. D-Cycloserin wird in einer Konzentration von 0,9 g in 18 ml dest. Wasser gelöst und anschließend über einen Sterilfilter mit einer Porengröße von 0,2 µm sterilfiltriert. Zu 100 ml Basismedium + Indikator (4-fach Konzentrat) werden 1,6 ml der Cycloserinlösung zugesetzt. Trimethoprim wird als Stocklösung in Dimethylsulfoxid (126 mg Trimethoprim auf 4,5 ml DMSO) hergestellt. Zu 100 ml fertigem Basismedium + Indikator (4-fach Konzentrat) werden 0,4 ml der Stocklösung zugesetzt.

### Durchführung des Nachweisverfahrens

Gemäß einer bevorzugten Ausführungform werden Standard-96 Well-Mikrotiterplatten verwendet. Bei diesen Platten sind in je 12 vertikalen Reihen je 8 Vertiefungen angeordnet. Die ersten 4 Reihen (32 Wells) werden dabei mit je 0,24 ml der Untersuchungsprobe (Milch oder in steriler Milch homogenisierte Käseproben, die davor durch Inkubation über 20 min bei 80°C pasteurisiert wurden) beschickt und je Well mit 0,08 ml eines sterilen Konzentrats des erfindungsgemäßen Nährmediums versehen.

In gleicher Weise werden die 32 Wells der Reihen 5-8 mit je 0,12 ml Probe bzw. Probenhomogenat und je 0,04 ml eines sterilen Konzentrats des erfindungsgemäßen Nährmediums sowie die 32 Wells der Reihen 9-12 mit je 0,06 ml Probe bzw. Probenhomogenat und mit je 0,02 ml des sterilen Konzentrats (4-fach) des Nährmediums versehen. Die Beschickung der Mikrotiterplatten mit Probe und Nährmedium kann dabei mit automatischen Pipetten oder vollautomatisch durch ein geeignetes Robotersystem für die Beschickung von Mikrotiterplatten erfolgen. Ebenso können Nährmedium und Probe bereits vor dem Pipettieren im Verhältnis 1:3 gemischt und gemeinsam in den oben angeführten Mengen und Abstufungen in die einzelnen Vertiefungen der Mikrotiterplatten pipettiert werden. Die Mikrotiterplatten werden anschließend bei 37°C unter anaeroben Bedingungen (10% CO₂, 10% H₂ und 80% N₂ Gemisch) für 48 Stunden inkubiert.

Je nach vorhandenem Kontaminationsgrad ergibt sich nach der Bebrütung in den Replikaten eine bestimmte Verteilung positiver und negativer Wells, welche über ihre Farbe (rot = negativ; gelb = positiv) oder über ein entsprechendes Fluoreszenzsignal (positiv) bzw. Fehlen des Fluoreszenzsignals (negativ) auswertbar sind. Mittels statistischer Berechnung lässt sich jeder möglichen Verteilung an bewachsenen und unbewachsenen Vertiefungen innerhalb der untersuchten Verdünnungsstufen eine wahrscheinlichste Keimzahl (MPN Sporenzahl) zuordnen.

Bei der Auswertung der einzelnen Vertiefungen werden die Farbumschläge (Rot nach Gelb) oder ein Fluoreszenzsignal detektiert und die "wahrscheinlichste Keimzahl" vorzugsweise über eine entsprechende Software zur Berechnung von MPN-Ergebnissen anhand der eingesetzten Probenverdünnungen und der Anzahl der verwendeten Replikate berechnet. Die Verwendung von 32 Replikaten pro Verdünnungsstufe sowie die geringe Abstufung der eingesetzten Konzentrationen (serielle 2-fach-Verdünnung) ermöglicht dabei eine sehr hohe Präzision der statistischen Berechnung.

Im oben beschriebenen Beispiel wurden insgesamt 13,44 ml Probe in 3 seriellen 2-fach-Verdünnungsstufen untersucht. Auf diese Weise konnten Sporenzahlen von 75 bis 59.000 Sporen pro Liter Milch verlässlich nachgewiesen werden.

Tabelle 4 zeigt einen Methodenvergleich bei der Untersuchung der bevorzugten Methode der gegenständlichen Erfindung mit der aktuell in der Routine häufig eingesetzten Methode nach Bryant und Burkey. Daraus ist ersichtlich, dass die Sporenzahlen nach Bryant und Burkey, bedingt durch die geringere Selektivität dieses Verfahrens, meist signifikant höher liegen als die mit dem erfindungsgemäßen Verfahren ermittelten tatsächlichen Sporenzahlen.

Zumindest für die Proben Nr. 5, 21, 24, 31 und 34 wurden dabei mittels molekularbiologischer Verfahren neben dem Nachweis von Clostridium tyrobutyricum auch das Vorhandensein von nicht zu den Zielorganismen zählenden Mikroorganismen, wie Staphylococcus pasteuri, Bacillus thuringensis, Bacillus toyonensis, Staphylococcus petrasii, Streptococcus mitis oder Clostridum subterminale, nachgewiesen. Diese Nicht-Zielorganismen sind in der Lage, bei der Methode nach Bryant und Burkey zu einem positiven Nachweis (Wachstum mit Gasbildung) zu führen. Es konnte gezeigt werden, dass diese Isolate beim erfindungsgemäßen Verfahren bzw. durch die Verwendung des erfindungsgemäßen Cycloserin und Trimethoprim enthaltenden Nährmediums im Wachstum unterdrückt werden und keinen Farbumschlag auslösen können.

**Tabelle 4: Vergleich der Ergebnisse bei der Untersuchung von 39 Rohmilchproben mit dem erfindungsgemäßen Verfahren und der Methode nach Bryant und Burkey.**

| **Probe** | **Mittelwert (erfindungsgemäßes Verfahren) nach 48 Stunden** | **Mittelwert (Bryant und Burkey) nach 7 Tagen** |
|---|---|---|
| 1 | 5485 | >16000 |
| 2 | 4063 | 9200 |
| 3 | 1170 | 2600 |
| 4 | 10698 | >16000 |
| 5 | 1587 | 2950 |
| 6 | 2860 | 10700 |
| 7 | 4980 | >16000 |
| 8 | 344 | 2050 |
| 9 | 7647 | 7300 |
| 10 | 6603 | 12600 |
| 11 | 114 | <180 |
| 12 | 7955 | >16000 |
| 13 | <75 | 315 |
| 14 | <75 | <180 |
| 15 | <75 | 495 |
| 16 | <75 | <180 |
| 17 | >58700 | >16000 |
| 18 | <75 | 190 |
| 19 | <75 | 190 |
| 20 | 5070 | 9200 |
| 21 | 550 | 1500 |
| 22 | 878 | 3250 |
| 23 | 6865 | 12600 |
| 24 | 9520 | >16000 |
| 25 | 353 | 495 |
| 26 | 113 | 620 |
| 27 | 1345 | 6350 |
| 28 | 2285 | 4450 |
| 29 | 1565 | 4450 |
| 30 | 466 | 2850 |
| 31 | 190 | 620 |
| 32 | 2310 | 10700 |
| 33 | 897 | 1075 |
| 34 | 3700 | 12600 |
| 35 | 1870 | 1245 |
| 36 | 1335 | 4450 |
| 37 | 4455 | 12600 |
| 38 | <30 | <180 |
| 39 | <30 | <180 |

### LITERATUR

- Abgrall B., Bourgeois C. and Bourva F. (1985) Lait 65, 45-53
- Batina P., Arnold F., Bedouet L., Robreau G., Talbot F., and Malcoste (1997) Journal of Applied Microbiology 82, 619-624
- Bergere J.L and Sivelä S. (1990) Bulletin of the International Dairy Federation, 18-23
- Blaschek H.P. (1999) Encyclopedia of Food Microbiology, 428-451
- Bourgeois C.M., Le Parc O., Abgrall B. and Clerte J. (1984) Journal of Dairy Science, 67, 2493-2499
- Brändle J., Domig J. and Kneifel W. (2016) Food Control 67, 96-113
- Hirsch A. and Grinstead E. (1954) J. Dairy. Res. 21. 101-110
- Jonsson A. (1990) Journal of Dairy Science 73, 719-725
- Julien M.C., Dion P., Lafreniere C., Antoun H. and Drouin P.(2008) Applied an Environmental Microbiology 74, 6348-6357
- Rainey F.A., Hollen B.J. and Small A. (2009) Bergey's Manual of Systematic Microbiology Vol. 3 The Firmicutes, 736-827
- Roux V. and Raoult D. (2004) International Journal of Systematic and Evolutionary Microbiology 5, 1049-1054
- Zangerl P. (1993), Deutsche Milchwirtschaft Vol. 19, 936-940

## Patentansprüche

1. Flüssiges Nährmedium für die Kultivierung käsereischädlicher Clostridien, enthaltend ein Reinforced Clostridial Medium (RCM), zur Inkubation mit Milch- oder Käseproben, **dadurch gekennzeichnet, dass** es Neutralrot als Indikator sowie Cycloserin und Trimethoprim umfasst.

2. Flüssiges Nährmedium nach Anspruch 1, **dadurch gekennzeichnet, dass** die Konzentration von Neutralrot bei der Inkubation 0,010 bis 0,025 g/l, vorzugsweise 0,015 g/l, beträgt.

3. Flüssiges Nährmedium nach Anspruch 1 oder 2, **dadurch gekennzeichnet, dass** die Konzentration von Trimethoprim bei der Inkubation 17 mg/l bis 35 mg/l, vorzugsweise 28 mg/l, beträgt.

4. Flüssiges Nährmedium nach einem der Ansprüche 1 bis 3, **dadurch gekennzeichnet, dass** die Konzentration von Cycloserin bei der Inkubation 200 mg/l beträgt.

5. Verfahren zum Nachweis von käsereischädlichen Clostridien in Milch nach der "Most Probable Number" (MPN) - Methode unter Verwendung eines flüssigen Nährmediums nach einem der Ansprüche 1-4, wobei die Milch mit dem Nährmedium inkubiert wird, **dadurch gekennzeichnet, dass** die Inkubation der Milch bei einem pH-Wert von 6,0-7,2, vorzugsweise 7,0, unter anaeroben Bedingungen durchgeführt und die Probe nach der Inkubation hinsichtlich einer Farbänderung ausgewertet wird.

6. Verfahren nach Anspruch 5, **dadurch gekennzeichnet, dass** das Nährmedium mit der Milch gemischt wird, wobei der Anteil der Milch >50 Vol.%, vorzugsweise 75%, beträgt.

7. Verfahren nach Anspruch 5, **dadurch gekennzeichnet, dass** die Inkubation mittels mehrfacher Replikate von seriellen Probenverdünnungen in Mikrotiterplatten durchgeführt und die Auswertung mittels Fluoreszenzmessung vorgenommen wird, wobei das Nährmedium mit der Milch in einem Volumenverhältnis Nährmedium:Milch = 9:1 oder größer gemischt wird.

## Claims

1. A liquid culture medium for the cultivation of clostridia harmful to the cheese-making industry, containing a reinforced clostridial medium (RCM), for the incubation with milk or cheese samples, **characterized in that** it comprises neutral red as an indicator as well as cycloserine and trimethoprim.

2. A liquid culture medium according to claim 1, **characterized in that** the concentration of neutral red upon incubation is 0.010 to 0.025 g/l, preferably 0.015 g/l.

3. A liquid culture medium according to claim 1 or 2, **characterized in that** the concentration of trimethoprim upon incubation is 17 mg/l to 35 mg/l, preferably 28 mg/l.

4. A liquid culture medium according to any of claims 1 to 3, **characterized in that** the concentration of cycloserine upon incubation is 200 mg/l.

5. A method for the detection in milk of clostridia harmful for the cheese-making industry, according to the "most probable number" (MPN) method using a liquid culture medium according to any of claims 1 to 4, wherein the milk is incubated with the culture medium, **characterized in that** the incubation of the milk is carried out at a pH value of 6.0-7.2, preferably 7.0, under anaerobic conditions and that the sample is evaluated following incubation in regard to a colour change.

6. A method according to claim 5, **characterized in that** the culture medium is mixed with the milk, wherein the proportion of the milk is >50% by volume, preferably 75%.

7. A method according to claim 5, **characterized in that** the incubation is carried out using multiple replicates of serial sample dilutions in microtiter plates and that the evaluation is performed using fluorescence measurement, wherein the culture medium is mixed with the milk at a volume ratio of culture medium : milk = 9:1 or higher.

## Revendications

1. Milieu de culture liquide pour la culture de clostridium nuisible pour l'industrie fromagère, contenant un milieu renforcé pour clostridium (MRC), pour l'incubation avec des échantillons de lait ou de fromage, **caractérisé en ce qu'**il comprend du rouge neutre comme indicateur ainsi que de la cyclosérine et du triméthoprime.

2. Milieu de culture liquide selon la revendication 1, **caractérisé en ce que** la concentration de rouge neutre lors de l'incubation vaut 0,010 à 0,025 g/l, de préférence 0,015 g/l.

3. Milieu de culture liquide selon une revendication 1 ou 2, **caractérisé en ce que** la concentration de triméthoprime lors de l'incubation vaut 17 mg/l à 35 mg/l, de préférence 28 mg/l.

4. Milieu de culture liquide selon l'une quelconque des revendications 1 à 3, **caractérisé en ce que** la concentration de cyclosérine lors de l'incubation vaut 200 mg/l.

5. Procédé de détection de clostridium nuisible pour l'industrie fromagère dans le lait selon la méthode "Most Probable Number" (MPN) [Nombre le plus probable] avec utilisation d'un milieu de culture liquide selon l'une quelconque des revendications 1 à 4, dans lequel on incube le lait avec le milieu de culture, **caractérisé en ce que** l'on effectue l'incubation du lait à un pH de 6,0-7,2, de préférence de 7,0, dans des conditions anaérobies et on évalue l'échantillon après l'incubation sous l'angle d'un changement de coloration.

6. Procédé selon la revendication 5, **caractérisé en ce que** l'on mélange le milieu de culture avec le lait, dans lequel la proportion de lait vaut > 50 % en volume, de préférence 75 %.

7. Procédé selon la revendication 5, **caractérisé en ce que** l'on effectue l'incubation au moyen de répliques multiples de dilutions d'échantillons en série dans des plaques de microtitrage et on procède à l'évaluation au moyen d'une mesure par fluorescence, dans lequel on mélange le milieu de culture avec le lait dans un rapport volumique milieu de culture : lait = 9 : 1 ou plus.
